(19) Europäisches Patentamt
European Patent Office
Office européen des brevets

(11) Publication number: **0 306 547 B1**

(12) **EUROPEAN PATENT SPECIFICATION**

(45) Date of publication of patent specification: **10.06.92**

(51) Int. Cl.5: **C07C 255/01**, C07D 213/61, C07C 253/30

(21) Application number: **87113211.4**

(22) Date of filing: **09.09.87**

(54) **2-Hydrocarbyl-3,6-dichloropyridines and their preparation.**

(43) Date of publication of application:
**15.03.89 Bulletin 89/11**

(45) Publication of the grant of the patent:
**10.06.92 Bulletin 92/24**

(84) Designated Contracting States:
**AT BE DE FR GB IT NL**

(56) References cited:
**EP-A- 0 012 117**
**EP-A- 0 137 415**
**DE-A- 2 423 316**

(73) Proprietor: **DOWELANCO**
**9002 Purdue Road**
**Indianapolis, Indiana 46268-3030(US)**

(72) Inventor: **Halpern, Marc E.**
**2905 Blairmont Drive**
**Midland Michigan 48640(US)**
Inventor: **Dietsche, Thomas J.**
**30 Roanoke Road**
**Berkeley California 94705(US)**
Inventor: **Orvik, Jon A.**
**1164 Lincoln Avenue no. 338**
**Walnut Creek California 94596(US)**
Inventor: **Barron, Brian J.**
**143 Dartmouth Place**
**Benicia California 94510(US)**

(74) Representative: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

3,6-Dichloropicolinic acid is a commercially useful herbicide (US-A-3 317 549) and 3,6-dichloro-2-(trichloromethyl)-pyridine is known as a nitrification inhibitor and as a herbicide (US-A-2 679 453 and US-A-3 135 594). These compounds are not readily obtained by the pyridine ring chlorination of a 2-substituted pyridine because such chlorinations are not sufficiently selective to produce a preponderance of the desired 3,6-dichloro-2-substituted pyridine isomer in the mixture obtained. Alternative methods for introducing chlorine into the pyridine nucleus selectively at the 3 and 6 positions of 2-substituted pyridines depend upon the presence of amino or hydroxyl groups at those positions in the starting materials and the requisite materials are not commercially available (Heterocyclic Compounds, Vol.14, "Pyridine and its Derivatives", Part 2).

It has now been found that 2-hydrocarbyl-3,6-dichloropyridines can be prepared by a ring closure method from readily available starting materials. A two step process is employed in which acrylonitrile and an appropriate dichloromethyl hydrocarbyl ketone are reacted under conditions conducive to the reaction to form a 1,1-dichloro-3-cyanopropyl hydrocarbyl ketone intermediate which is cyclized to obtain the desired 2-hydrocarbyl-3,6-dichloropyridines.

In the process of the present invention acrylonitrile is first treated with an appropriate dichloromethyl hydrocarbyl ketone of Formula I in the presence of a base, such as an alkali metal alkoxide or hydroxide or a tertiary amine, to obtain a 1,1-dichloro-3-cyanopropyl hydrocarbyl ketone of Formula II. The reaction can be illustrated as follows:

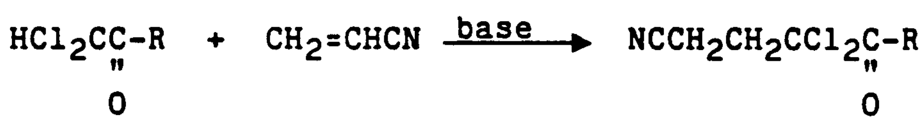


I II

wherein
R represents $C_1$-$C_8$ alkyl, $C_3$-$C_8$ cycloalkyl, or $C_4$-$C_8$ cycloalkylalkyl.

The 1,1-dichloro-3-cyanopropyl hydrocarbyl ketones of Formula II are then cyclized with hydrogen chloride to obtain 2-hydrocarbyl-3,6-dichloropyridines of Formula III. The reaction can be illustrated as follows:

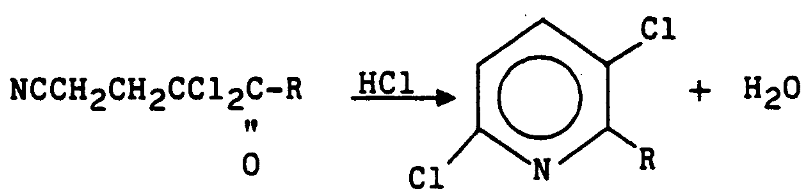


II III

wherein R is as hereinbefore defined.

Both the intermediate 1,1-dichloro-3-cyanopropyl hydrocarbyl ketones or Formula II and the product 2-hydrocarbyl-3,6-dichloropyridines of Formula III are novel compounds.

The term hydrocarbyl as used herein is meant to designate the following moieties: alkyl including straight and branched chain isomers, cycloalkyl including those having alkyl substituents (e.g. 2-methylcyclopropyl), and cycloalkylalkyl, such as cyclopentylmethyl.

The addition reaction of a dichloromethyl hydrocarbyl ketone with acrylonitrile according to the present process is typically carried out in an organic solvent, such as, for example, t-butanol, ethanol, dimethylformamide, dimethyl sulfoxide, acetonitrile, methylene chloride, tetrahydrofuran and toluene. Reaction temperatures of from 0 to 120°C, preferably from 40 to 90°, are normally employed. The reaction mixture is usually agitated and it is often convenient to carry out the reaction at its reflux temperature.

Suitable bases for the addition reaction are those that are capable of abstracting a proton from the dichloromethyl hydrocarbyl ketone and include alkali metal hydroxides, such as sodium hydroxide or potassium hydroxide; alkali metal alkoxides, such as potassium t-butoxide or sodium ethoxide; and trialkylamines, such as triethylamine, N,N-dimethyl-N-hexylamine, N,N̄,N′,N′-tetramethylethylenediamine, or N-methylpyrrolidine. When the base is an alkali metal hydroxide or alkali metal alkoxide, a quaternary ammonium salt, such as N,N,N-tricapryl-N-methylammonium chloride or N-benzyl-N,N,N-triethylammonium chloride, may be added to facilitate the reaction.

Approximately equimolar quantities of acrylonitrile and the dichloromethyl hydrocarbyl ketone or an excess of acrylonitrile can be conveniently employed in the process. The reaction is continued until a substantial amount of the desired 1,1-dichloro-3-cyanopropyl hydrocarbyl ketone product has formed or until one of the starting materials has been substantially depleted. The exact time will depend on the starting dichloromethyl hydrocarbyl ketone employed as well as the solvent and the reaction temperature used.

The 1,1-dichloro-3-cyanopropyl hydrocarbyl ketones of Formula II prepared in the above described procedures can be recovered using conventional means, such as, for example, distillation, extraction, chromatography and crystallization. After recovery of the 1,1-dichloro-3-cyanopropyl hydrocarbyl ketones in a pure or partially purified form, they may be utilized in the cyclization reaction of the invention.

The cyclization reaction of 1,1-dichloro-3-cyanopropyl hydrocarbyl ketones is accomplished by heating these compounds in the presence of hydrogen chloride. The hydrogen chloride can be added to the reaction medium all at once or continuously during the reaction period. Metal chloride Lewis acid catalysts, such as zinc chloride and aluminum chloride can be employed along with the hydrogen chloride to facilitate this reaction. The reaction generates water and this may be removed as it forms by distillation, absorption, or reaction. Generally, anhydrous reactants are employed.

The cyclization reaction can be carried out neat or in the presence of an organic solvent, such as, for example, acetic acid, dimethylformamide, dimethyl sulfoxide, dioxane, dimethoxyethane, methylene chloride and toluene. Reaction temperatures of 50° to 200°C and pressures of 1 to 5 atmospheres (101.325 to 506.625 kPa) are advantageously employed.

The reaction is continued until a substantial amount of the 2-hydrocarbyl-3,6-dichloropyridine product is formed or until the 1,1-dichloro-3-cyanopropyl hydrocarbyl ketone reactant is substantially depleted. The time required will vary depending upon the identity of the 1,1-dichloro-3-cyanopropyl hydrocarbyl ketone, the solvent, the concentration of hydrogen chloride and any Lewis acid catalysts, and the temperature employed.

The product 2-hydrocarbyl-3,6-dichloropyridines of Formula III can be removed from the reaction medium by conventional means, such as, for example, distillation, extraction and chromatography.

Examples of dichloromethyl hydrocarbyl ketones useful as starting materials, 1,1-dichloro-3-cyanopropyl hydrocarbyl ketones obtained as intermediates, and 2-hydrocarbyl-3,6-dichloropyridines obtained as products in the present invention include those compounds of Formulas I, II, and III wherein R represents, for example, methyl, ethyl, propyl, 1-methylethyl, 1,1-dimethylethyl, butyl, hexyl, cyclohexyl, cyclopentyl, cyclooctyl, cyclopentylmethyl and cyclopropylmethyl. Compounds of Formulas I, II and III wherein R represents $C_1$-$C_4$ alkyl, $C_3$-$C_6$ cycloalkyl, or $C_4$-$C_6$ cycloalkylalkyl constitute a preferred class.

The following examples illustrate the present invention.

Example 1 Preparation of 4,4-dichloro-5-oxo-hexanenitrile

Procedure A:

A mixture of 11 ml of t-butanol, 3 ml (31 mmol) of 1,1-dichloro-2-propanone and 2 ml (32 mmol) of acrylonitrile was placed in a 50 ml 3-necked round bottom flask equipped with a magnetic stirrer, a dropping funnel, a sampling port and a Y-tube fitted with a thermometer and an outlet to a scrubber. Four ml of 25 percent NaOH were added dropwise over 7 minutes, during which time the reaction exothermed to a final temperature of 71°C. After 68 minutes, with stirring, gas chromatographic (GC) analysis of the reaction mixture showed 40 percent unreacted 1,1-dichloro-2-propanone, 42 percent 4,4-dichloro-5-oxo-hexanenitrile, 10 percent 1,1-dichloro-2,4-cyclohexanedione, 2 percent 1-chloro-1-acetyl-2-cyanocyclopropane, and 1 percent 1,1,3,3-tetrachloro-2-methyl-4-oxo-2-pentanol.

An authentic sample of 4,4-dichloro-5-oxo-hexanenitrile, which was isolated by fractional distillation (b.p. 92°-95°C at 13 Pa (0.1 mm Hg) pressure) and purified by fractional crystallization (m.p. 49°-51°C),

analyzed as follows:

| Analysis | | | | |
|---|---|---|---|---|
| | % C | % H | % N | % Cl |
| Calc. for $C_6H_4Cl_2NO$:<br>Found: | 40.03<br>40.06 | 3.92<br>3.82 | 7.78<br>8.28 | 39.39<br>39.76 |
| NMR ($CDCl_3$): δ2.56 (s, 3H); δ2.72 (s, 4H) | | | | |

Procedure B:

A 3.99 g (31.4 mmol) portion of 1,1-dichloro-2-propanone was dissolved in 8 ml of t-butanol and the mixture heated to 40 °C. A solution of 1.61 g (30.3 mmol) of acrylonitrile in 3 ml of t-butanol and 10 ml of a 30 percent potassium hydroxide in methanol solution were added dropwise over 11 minute and 2.5 hour periods, respectively. The reaction was slow at first and the temperature fell to 25 °C. The mixture was mildly warmed and after the bulk of the potassium hydroxide was added the reaction became exothermic and the temperature rose to a maximum of 75 °C. After 4 hours the product mixture contained 40 percent 4,4-dichloro-5-oxo-hexanenitrile and 22 percent 1,1-dichloro-2-propanone by GC analysis.

Procedure C:

A solution containing 8.0 g (63 mmol) of 1,1-dichloro-2-propanone and 3.2 g (61 mmol) of acrylonitrile and 0.73 g (7.2 mmol) of triethylamine in 20 ml of ethanol was prepared and heated to 56 °C over a 21 hour period. Another 1.46 g (14.4 mmol) of triethylamine was then added and the reaction continued for 3 additional hours. The reaction product was found to contain 17 percent 4,4-dichloro-5-oxo-hexanenitrile and 43 percent 1,1-dichloro-2-propanone by GC analysis.

Example 2 Preparation of 3,6-dichloro-2-methylpyridine

Procedure A:

One g of 4,4-dichloro-5-oxo-hexanenitrile was placed in a 2-necked 10 ml pear shaped flask equipped with an HCl inlet and a Y-shaped tube attached to a NaOH scrubber and to a nitrogen inlet and holding a thermometer (touching the bottom of the flask). The flask was immersed in a silicone oil bath. HCl gas was bubbled through the 4,4-dichloro-5-oxo-hexanenitrile for 120 minutes during which time the temperature was maintained at 145-160 °C. A white solid condensate weighting 480 mg appeared in the Y-tube. GC analysis of the white condensate showed it to consist of about 50 percent 3,6-dichloro-2-methylpyridine and about 50 percent of a very high boiling material. GC analysis of the residue in the reaction flask showed it to consist of about 75 percent 4,4-dichloro-5-oxo-hexanenitrile and about 25 percent 3,6-dichloro-2-methylpyridine.

An authentic sample of 3,6-dichloro-2-methylpyridine, which was isolated by fractional distillation (b.p. 95 °C at 2,7 KPa (20 mm Hg) pressure), analyzed as follows:

| Analysis | | | |
|---|---|---|---|
| | % C | % H | % N |
| Calc. for $C_6H_5Cl_2N$:<br>Found: | 44.5<br>44.9 | 3.1<br>3.2 | 8.6<br>9.0 |
| NMR ($CDCl_3$): δ2.60 (s, 3H), δ7.17 (d-7.6 Hz, 1H), δ7.64 (d-7.6 Hz, 1H) | | | |

Procedure B:

A 1.11 g sample of 4,4-dichloro-5-oxo-hexanenitrile was placed in an 8 oz pressure bottle and

pressurized to 102.6 KPa (15 psi) with hydrogen chloride gas. The bottom portion of the bottle was heated in a silicone oil bath to a maximum of 197°C for 30 minutes and at 110-155° for another 6 hours. After cooling the reaction mixture was diluted with methanol, filtered, basified and extracted with methylene chloride to obtain a product that was 45 percent 3,6-dichloro-2-methylpyridine by GC analysis.

Acrylonitrile is an item of commerce and readily available. Dichloromethyl hydrocarbyl ketones of Formula I are generally known in the art. They can be prepared from the corresponding methyl hydrocarbyl ketones by the formation of an imino derivative and subsequent chlorination of that derivative with N-chlorosuccinimide as described in Bull. Soc. Chim. Belg., 81, 643-7 (1972).

## Claims

**1.** Process for preparing 1,1-dichloro-3-cyanopropyl hydrocarbyl ketone compounds of the formula

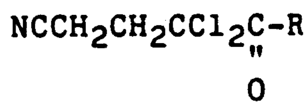

wherein R represents $C_1$-$C_8$ alkyl, $C_3$-$C_8$ cycloalkyl, or $C_4$-$C_8$ cycloalkylalkyl, characterized by reacting, in the presence of a base, acrylonitrile and a dichloromethyl hydrocarbyl ketone of the formula

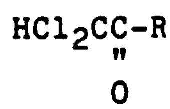

wherein R is as defined before, under conditions conducive to the reaction and, thereafter, recovering the product compound.

**2.** Process of Claim 1 wherein the base is a trialkylamine.

**3.** Process of Claim 1 wherein the base is an alkali metal $C_1$-$C_4$ alkoxide.

**4.** Process of Claim 1 wherein the base is an alkali metal hydroxide.

**5.** Process of Claim 1 wherein R is $C_1$-$C_4$ alkyl, $C_3$-$C_6$ cycloalkyl or $C_4$-$C_6$ cycloalkylalkyl.

**6.** Process of Claim 5 wherein R is methyl.

**7.** A process for preparing a 2-hydrocarbyl-3,6-dichloropyridine compound of the formula

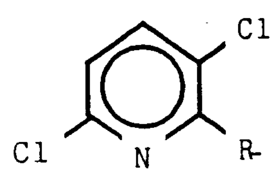

wherein R represents $C_1$-$C_8$ alkyl, $C_3$-$C_8$ cycloalkyl, or $C_4$-$C_8$ cycloalkylalkyl, which is characterized by acidifying a 1,1-dichloro-3-cyanopropyl hydrocarbyl ketone compound of the formula

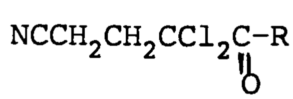

wherein R is defined as before with hydrogen chloride, heating the acidified intermediate, and thereafter, recovering the 2-hydrocarbyl-3,6-dichloropyridine compound.

**8.** A 1,1-dichloro-3-cyanopropyl hydrocarbyl ketone compound of the formula

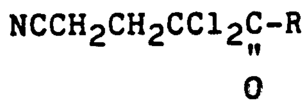

wherein R represents $C_1$-$C_8$ alkyl, $C_3$-$C_8$ cycloalkyl, or $C_4$-$C_8$ cycloalkylalkyl.

**9.** A compound of Claim 8 wherein R is $C_1$-$C_4$ alkyl, $C_3$-$C_6$ cycloalkyl, or $C_4$-$C_6$ cycloalkylalkyl.

**10.** A compound of Claim 9 wherein R is methyl.

**11.** A 2-hydrocarbyl-3,6-dichloropyridine compound of the formula

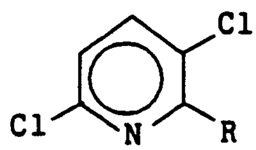

wherein R represents $C_1$-$C_8$ alkyl, $C_3$-$C_8$ cycloalkyl, or $C_4$-$C_8$ cycloalkylalkyl.

**12.** A compound as claimed in Claim 11 wherein R is $C_1$-$C_4$ alkyl, $C_3$-$C_6$ cycloalkyl, or $C_4$-$C_6$ cycloalkylalkyl.

**13.** A compound as claimed in Claim 12 wherein R is methyl.

**Revendications**

**1.** Procédé de préparation de composés 1,1-dichloro-3-cyanopropylhydrocarbylcétones de formule

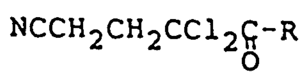

dans laquelle R représente un groupe alkyle en $C_1$-$C_8$, cycloalkyle en $C_3$-$C_8$ ou cycloalkylalkyle en $C_4$-$C_8$, caractérisé en ce que l'on fait réagir, en présence d'une base, de l'acrylonitrile et une dichlorométhylhydrocarbylcétone de formule

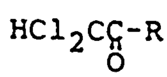

dans laquelle R est tel que défini ci-dessus, dans des conditions favorables à la réaction et, ensuite, on récupère le composé produit.

**2.** Procédé selon la revendication 1, dans lequel la base est une trialkylamine.

**3.** Procédé selon la revendication 1, dans lequel la base est un alcoxyde en $C_1$-$C_4$ de métal alcalin.

**4.** Procédé selon la revendication 1, dans lequel la base est un hydroxyde de métal alcalin.

**5.** Procédé selon la revendication 1, dans lequel R est un groupe alkyle en $C_1$-$C_4$, cycloalkyle en $C_3$-$C_6$

ou cycloalkylalkyle en $C_4$-$C_6$.

6. Procédé selon la revendication 5, dans lequel R est un groupe méthyle.

7. Procédé de préparation d'un composé 2-hydrocarbyl-3,6-dichloropyridine de formule

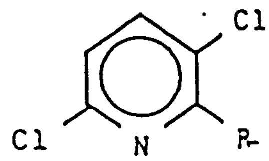

dans laquelle R représente un groupe alkyle en $C_1$-$C_8$, cycloalkyle en $C_3$-$C_8$ ou cycloalkylalkyle en $C_4$-$C_8$, qui est caractérisé en ce que l'on acidifie un composé 1,1-dichloro-3-cyanopropylhydrocarbylcétone de formule

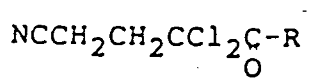

dans laquelle R est tel que défini ci-dessus, avec du chlorure d'hydrogène, on chauffe l'intermédiaire acidifié et, ensuite, on récupère le composé 2-hydrocarbyl-3,6-dichloropyridine.

8. Composé 1,1-dichloro-3-cyanopropylhydrocarbylcétone de formule

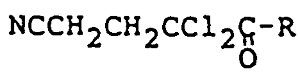

dans laquelle R représente un groupe alkyle en $C_1$-$C_8$, cycloalkyle en $C_3$-$C_8$ ou cycloalkylalkyle en $C_4$-$C_8$.

9. Composé selon la revendication 8, dans lequel R est un groupe alkyle en $C_1$-$C_4$, cycloalkyle en $C_3$-$C_6$ ou cycloalkylalkyle en $C_4$-$C_6$.

10. Composé selon la revendication 9, dans lequel R est un groupe méthyle.

11. Composé 2-hydrocarbyl-3,6-dichloropyridine de formule

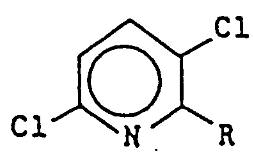

dans laquelle R représente un groupe alkyle en $C_1$-$C_8$, cycloalkyle en $C_3$-$C_8$ ou cycloalkylalkyle en $C_4$-$C_8$.

12. Composé selon la revendication 11, dans lequel R est un groupe alkyle en $C_1$-$C_4$, cycloalkyle en $C_3$-$C_6$ ou cycloalkylalkyle en $C_4$-$C_6$.

13. Composé selon la revendication 12, dans lequel R est un groupe méthyle.

**Patentansprüche**

1. Verfahren zur Herstellung von 1,1-Dichlor-3-cyanopropylhydrocarbylketon-Verbindungen der Formel

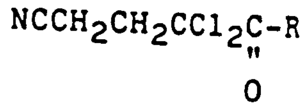

worin R $C_1$-$C_8$ Alkyl, $C_3$-$C_8$ Cycloalkyl oder $C_4$-$C_8$ Cycloalkylalkyl darstellt, **dadurch gekennzeichnet,** daß man in Gegenwart einer Base Acrylnitril und ein Dichlormethylhydrocarbylketon der Formel

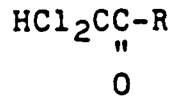

worin R wie zuvor definiert ist, unter reaktionsbegünstigenden Bedingungen umsetzt und danach die Produktverbindung gewinnt.

**2.** Verfahren nach Anspruch 1, worin die Base ein Trialkylamin ist.

**3.** Verfahren nach Anspruch 1, worin die Base ein Alkalimetall-$C_1$-$C_4$-alkoxid ist.

**4.** Verfahren nach Anspruch 1, worin die Base ein Alkalimetallhydroxid ist.

**5.** Verfahren nach Anspruch 1, worin R $C_1$-$C_4$ Alkyl, $C_3$-$C_6$ Cycloalkyl oder $C_4$-$C_6$ Cycloalkylalkyl ist.

**6.** Verfahren nach Anspruch 5, worin R Methyl ist.

**7.** Verfahren zur Herstellung einer 2-Hydrocarbyl-3,6-dichlorpyridin-Verbindung der Formel

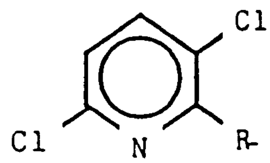

worin R $C_1$-$C_8$ Alkyl, $C_3$-$C_8$ Cycloalkyl oder $C_4$-$C_8$ Cycloalkylalkyl darstellt, **dadurch gekennzeichnet,** daß man eine 1,1-Dichlor-3-cyanopropylhydrocarbylketon-Verbindung der Formel

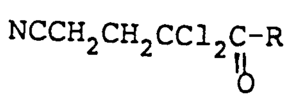

worin R wie zuvor definiert ist, mit Chlorwasserstoff ansäuert, das angesäuerte Zwischenprodukt erhitzt und danach die 2-Hydrocarbyl-3,6-dichlorpyridin-Verbindung gewinnt.

**8.** 1,1-Dichlor-3-cyanopropylhydrocarbylketon-Verbindung der Formel

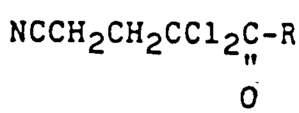

worin R $C_1$-$C_8$ Alkyl, $C_3$-$C_8$ Cycloalkyl oder $C_4$-$C_8$ Cycloalkylalkyl darstellt.

**9.** Verbindung nach Anspruch 8, worin R $C_1$-$C_4$ Alkyl, $C_3$-$C_6$ Cycloalkyl oder $C_4$-$C_6$ Cycloalkylalkyl ist.

**10.** Verbindung nach Anspruch 9, worin R Methyl ist.

**11.** 2-Hydrocarbyl-3,6-dichlorpyridin-Verbindung der Formel

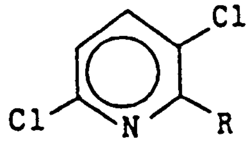

worin R $C_1$-$C_8$ Alkyl, $C_3$-$C_8$ Cycloalkyl oder $C_4$-$C_8$ Cycloalkylalkyl darstellt.

**12.** Verbindung nach Anspruch 11, worin R $C_1$-$C_4$ Alkyl, $C_3$-$C_6$ Cycloalkyl oder $C_4$-$C_6$ Cycloalkylalkyl ist.

**13.** Verbindung nach Anspruch 12, worin R Methyl ist.